# EUROPEAN PATENT SPECIFICATION

(11) **EP 4 622 589 B1**
(45) Date of publication and mention of the grant of the patent: **14.10.2026**
(21) Application number: 23822451.3
(22) Date of filing: 22.11.2023
(51) Int. Cl.: A61C 11/00, A61C 19/045

(54) **APPARATUS FOR SIMULATING THE MOVEMENTS OF THE TEMPOROMANDIBULAR JOINT**
VORRICHTUNG ZUR SIMULATION DER KIEFERGELENKSBEWEGUNGEN
APPAREIL POUR SIMULER LES MOUVEMENTS DE L'ARTICULATION TEMPOROMANDIBULAIRE

(30) Priority: 22.11.2022 IT 202200024039
(43) Date of publication of application: 01.10.2025
(73) Proprietor: Rampulla, Giuseppe, 43028 Tizzano Val Parma (PR) (IT); Pighin, Luca, 33080 Zoppola (PN) (IT)
(72) Inventor: Rampulla, Giuseppe, 43028 Tizzano Val Parma (PR) (IT); Pighin, Luca, 33080 Zoppola (PN) (IT)
(74) Representative: Fisauli, Beatrice A. M.
(86) International application number: PCT/IB2023/061776
(87) International publication number: WO 2024/110885

(56) References cited:
- US-A- 4 836 778
- KIM DAE-SEUNG ET AL: "Principal direction of inertia for 3D trajectories from patient-specific TMJ movement", COMPUTERS IN BIOLOGY AND MEDICINE, NEW YORK, NY, US, vol. 43, no. 3, 12 January 2013 (2013-01-12), pages 169 - 175, XP028577947, ISSN: 0010-4825, DOI: 10.1016/J.COMPBIOMED.2012.12.007

## Description

The present invention relates to an apparatus for simulating the biomechanics of movements of the temporomandibular joint. More in detail, the invention relates to an apparatus configured to simulate the effects of modifications and alterations of the three-dimensional trajectories of a patient's real mandibular kinematics.

The study of the movements of the temporomandibular joint and their reconstruction/simulation are particularly useful both for the dentist and for the dental technician to create rehabilitative medical devices, such as bridges, bites or the like, which, in turn, are used by the dentist to functionally or aesthetically restore a patient's teeth.

For this reason, various methods and related devices have been developed to track the movements of a person's jaw, in particular chewing, in order to obtain a digital model which may be used to verify the correct shape and size of a rehabilitation device, such as for example a dental prosthesis or other.

WO 2020/222162 A1 discloses an apparatus for recording and tracking the movements of a person's jaw based on a vision system and a processing algorithm capable of extrapolating three-dimensional trajectories of jaw movement.

Another document in the art is exemplified by KIM DAE-SEUNG ET AL: "Principal direction of inertia for 3D trajectories from patient-specific TMJ movement",COMPUTERS IN BIOLOGY AND MEDICINE, NEW YORK, NY, US, vol. 43, no. 3, 12 January 2013 (2013-01-12), pages 169-175, ISSN: 0010-4825, DOI: 10.1016/J.COMPBIOMED.2012.12.007.

A typical output of the aforementioned apparatus includes a three-dimensional graphic representation, i.e. in the three components carried out on the three anatomical planes of space: sagittal, frontal and transverse.

This apparatus can interface both with CAD modelling software and with known devices configured to replicate and physically simulate the movements of the temporomandibular joint.

However, expert dental professionals need to plan and evaluate in advance the effects induced by their interventions on joint biomechanics.

In the prior art, there are no known solutions that allow simulating these effects, starting from real mandibular kinematics, for the purpose of designing the rehabilitation treatment, such as the creation of prostheses.

In fact, all rehabilitation treatments to date are aimed at restoring masticatory function and aesthetics, without taking into account quantitative and functional parameters of the temporomandibular joint.

WO 2012/006696 A1 and EP 1178760 A1 describe methods and devices that allow simulating the movements of the jaw which, however, are obtained from databases or libraries of data representative of the movement of the jaw and not from real data of a specific patient.

In this context, the object of the present invention is to propose an apparatus for simulating the movements of the temporomandibular joint, which overcomes the drawbacks of the prior art.

In detail, it is the object of the invention to propose an apparatus that allows a set of real data representing the movements of a patient's jaw to be modified and a set of modified data that simulate the effects of a corrective intervention or a prosthetic apparatus on these movements to be generated.

A further object of the invention is to propose a practical and intuitive apparatus to use, which allows an operator to carry out modification of real data and simulation in a simple and fast manner.

The object of the present invention is also to propose an apparatus that allows the management of data representing movement in a three-dimensional space.

These specified objects are substantially achieved by an apparatus for simulating the movements of the temporomandibular joint according to claim 1.

In detail, the apparatus comprises at least a computer system and a simulator device. The simulator device is connected to the computer system and is configured to generate a simulation of jaw movement.

The computer system may typically comprise the hardware of a computer or a mobile device such as a tablet or the like. The computer system therefore generally comprises at least a programmable processor, a memory unit readable by the processor, a display device, for example a screen or a display, and an input device, for example a pointing device, a keyboard or a touch screen.

The computer system may further comprise a computer program for providing instructions to the processor. Alternatively, the program and its instructions may be implemented in machine language, if desired.

The simulator device may comprise a device for replicating the movement of the temporomandibular joint of a known type, either of a software type, for example a CAD software dedicated to dental applications, or a robotic device, such as for example those described in WO2013178828A1, IT102017093992, WO2020007507A1 or CN102626348A.

According to the invention, the computer system is configured to generate data sets representative of the actual movement of a person's jaw.

The simulator device is therefore configured to receive from the computer system a data set representative of a movement of a person's jaw and to generate a simulation of said movement.

The acquisition of real data of the kinematics of the person's temporomandibular joint may be carried out, for example, with devices based on optical systems, such as the one described in WO 2020/222162 A1 or with other equivalent devices also based on optical systems, or other systems.

According to an embodiment of the invention, said apparatus for acquiring data representative of the real movement of the jaw is included in the apparatus of the present invention.

Said acquired data is typically in the form of a set of Cartesian coordinates xₙ-yₙ, xₙ-zₙ, yₙ-zₙ on at least one, two or, preferably, three Cartesian reference planes (X-Y, X-Z, Y-Z). Each plane generally corresponds to one of the anatomical planes among sagittal, frontal and transverse.

More precisely, said data includes, for each Cartesian plane, at least a number N of points with coordinates xₙ-yₙ, xₙ₋z, yₙ-zₙ.

According to a variant, the computer system may also be configured to process the data acquired by the acquisition apparatus and to transform it in the form of Cartesian coordinates.

According to the invention, the computer system comprises at least one display device (screen, display, etc.) configured to generate, starting from the set of data representing the movement of a person's jaw in the form of a set of Cartesian coordinates, a computerised graphic representation on at least one Cartesian plane X-Y, X-Z, Y-Z of a movement trajectory of at least one reference point of the jaw. Preferably the display device is configured to generate a graphic representation of said movement trajectory for each Cartesian plane X-Y, X-Z, Y-Z.

According to a preferred embodiment, said reference point may be the centre of gravity of the jaw, which, for example, is calculated by the acquisition system mentioned above. Said reference point may, however, be different, for example it may be located on one of the person's teeth.

According to other variants of the invention, the reference points may also be two, three or more.

The kinematic trajectories acquired through the acquisition system are preferably represented graphically as lines joining points with three-dimensional coordinates xₙ-yₙ-zₙ with respect to a reference system X, Y, Z.

According to the invention, the graphic representation therefore comprises N points of coordinates xₙ-yₙ, xₙ-zₙ, yₙ-zₙ shown on a respective Cartesian plane X-Y, X-Z, Y-Z and at least one line representing an interpolation function of said N points. The interpolation function is calculated by the computer system, specifically by the processor.

Said interpolation function is preferably a spline, more preferably a cubic spline.

The graphic representation of the real data acquired on the person represents the starting condition from which it is possible to make changes to the movement trajectories, as illustrated below.

According to a variant of the invention, the display device may be configured to generate a graphic representation of movement trajectories in a three-dimensional space X-Y-Z.

Advantageously, according to the invention, the computer system in fact comprises at least one input device configured to impart, through the display device, a manual command to move at least one point (xₙ-yₙ, xₙ-zₙ, yₙ-zₙ) on at least one Cartesian axis X-Y, X-Z, Y-Z.

According to a possible preferred variant, the input device may comprise a mouse, a track pad or an equivalent device, which, through software implemented in the computer system, controls a pointer shown on the display device.

Alternatively, said input device is integrated into the display device in the form of a touch screen or the like.

Using the input device it is therefore possible to modify the coordinates of one or more points of the trajectory. According to a preferred embodiment, modifying the coordinates of a point in a Cartesian plane may be carried out by dragging the point with the input device, for example with the mouse pointer or with the finger, directly on the touch screen.

The computer system is therefore configured to recalculate the new coordinates of the modified point, i.e. moved on the Cartesian plane, and to recalculate a new interpolation function of the N points of updated coordinates.

The display device is therefore configured to show, on the relevant Cartesian plane, and possibly in three-dimensional space, the updated position of all the points and the new interpolation line representing the movement trajectory.

According to the invention, the computer system is further configured to send to the simulator device a data set representative of the real movement of a person's jaw corresponding to or based on the coordinates of the N points of the movement trajectory.

In this way a dentist (or another dental professional) may apply, in a simple and particularly intuitive manner, corrections to real movement trajectories that simulate corrective interventions to/on the movement of the temporomandibular joint.

These corrections may then be studied and evaluated in real time by virtue of the simulation of the correct movement provided by the simulator device.

The particularly practical way of modifying the movement trajectory allows the dentist to carry out numerous correction interactions in particularly short times.

Furthermore, the graphic representation of the real (original) and modified trajectories helps the professional operator to evaluate the extent of the correction to be made even before the simulation, reducing the number of iterations necessary to reach the optimal condition.

Other features and advantages will become apparent in the indicative, and therefore non-limiting, description of an example of a preferred, but non-exclusive, embodiment of the invention, as illustrated in the accompanying figures in which:
- Figure 1 is a schematic representation of the apparatus according to the invention;
- Figure 2 is a flow diagram representative of the use of the apparatus according to the invention;
- Figure 3 is a representation of the three-dimensional representation of the movement trajectories of some reference points of the jaw, displayed on the display device;

- Figure 4 is a representation of respective representations on three Cartesian planes of the movement trajectories of a reference point of the jaw, displayed on the display device;
- Figure 5 is a representation of the representations of Figure 3 according to another display mode;
- Figures 6a to 6c are representations of a detail of Figure 5, respectively before, during and after the modification of a point of the movement trajectory.

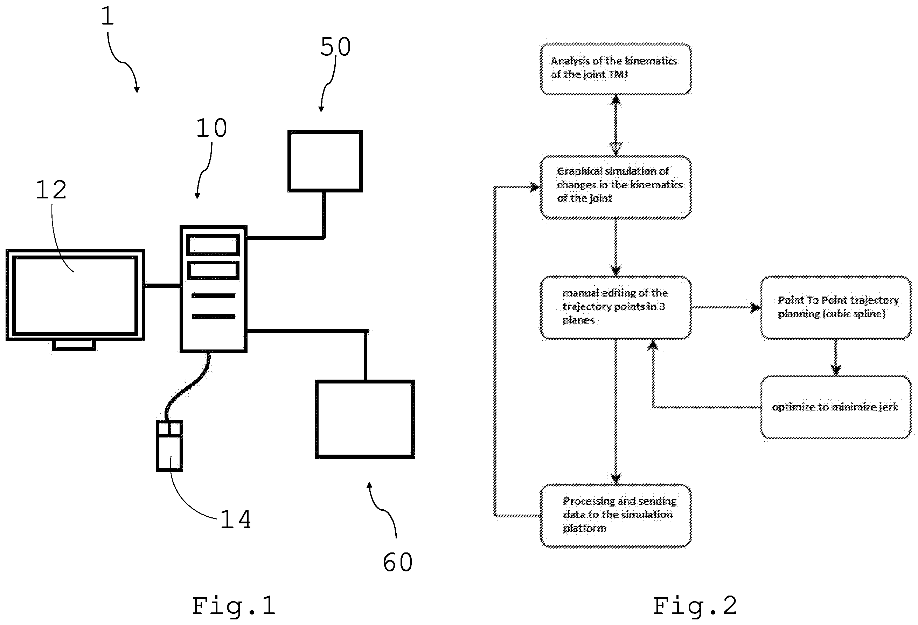

With reference to the accompanying Figure 1, the apparatus for simulating the movements of the temporomandibular joint, indicated as a whole with reference numeral 1, comprises a computer system 10, which in turn comprises a display device 12 and an input device 14.

The apparatus 1 is operationally connected with an apparatus 50 for recording the movements of the temporomandibular joint of a person and with a simulator device 60 for generating a simulation of the movement of the jaw.

Said apparatus 50 for recording the movements of the jaw or said simulator device 60 or both, may complete the apparatus 1 according to the present invention, i.e. they may be included in said apparatus.

With reference to the accompanying Figure 2, there is described the process, according to the present invention, to be able to carry out a simulation of the movement of a person's temporomandibular joint, in particular to simulate the effect of a corrective intervention or of a dental prosthetic device applied to the person.

This process involves a first stage consisting of recording the actual movements of a person's temporomandibular joint. As mentioned above, said recording step may be performed with an apparatus such as that described in WO 2020/222162 A1 or with other apparatus with optical vision devices or other systems.

The data generated by the recording apparatus 50 is provided to the computer system 10 of the apparatus 1 of the present invention.

The data provided by the recording apparatus 50 generally comprise three-dimensional coordinates xₙ-y ₙ-z ₙ of N points or said data may already be in the form of a set of Cartesian coordinates xₙ-yₙ, xₙ-zₙ, yₙ-zₙ of N points on three Cartesian planes X-Y, X-Z, Y-Z.

In the first case, the computer system 10 of the apparatus 1 of the invention may convert the data provided by the recording device 50 into such second form.

This data is defined as the data set D representing the person's jaw movement.

More in detail, the N points recorded describe the movement of at least one reference point of the jaw, typically at least the centre of gravity, during a pre-established movement, for example an opening and tightening cycle of the same.

The data set D may then be sent, from the computer system, to the simulator device 60 which generates a real simulation, in the case of a robotic device, or a virtual one, in the case of dental CAD software, of the real movement of the person's jaw.

This simulation allows the dentist to observe the real situation of the person's temporomandibular joint and to evaluate any corrective interventions whose effects may be simulated with the apparatus of the present invention, as described in detail below.

The computer system 10, starting from the N points of coordinates xₙ-yₙ, xₙ-zₙ, yₙ-zₙ and/or three-dimensional coordinates xₙ-yₙ-zₙ, calculates lines representative of an interpolation function of said N points.

In a subsequent, optional stage, the display device 12 shows a graphic representation in a three-dimensional system X-Y-Z of the movement trajectory of at least one reference point of the jaw.

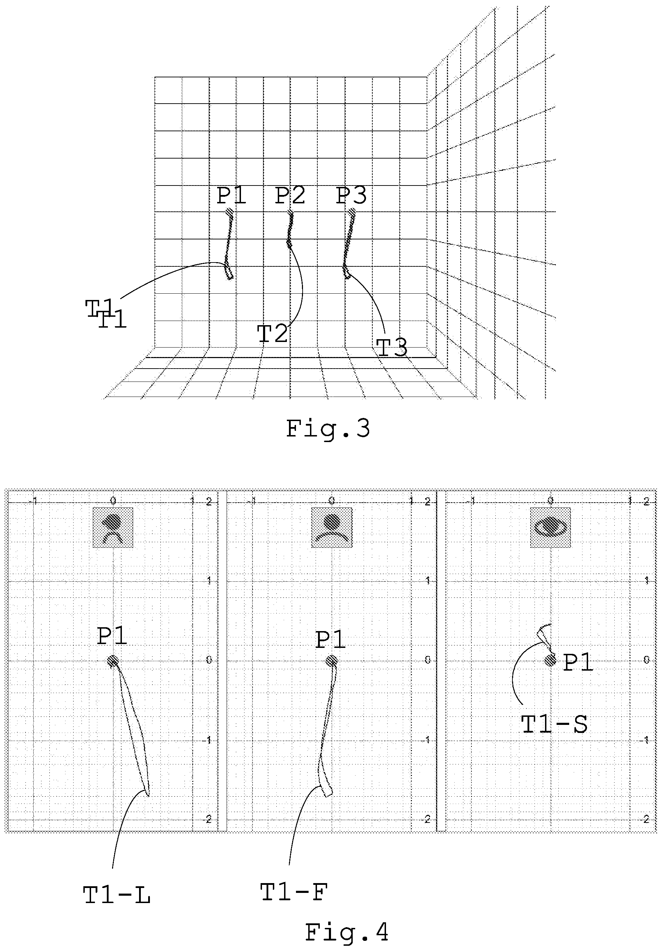

The accompanying Figure 3 shows an example of a graphic representation shown by the display device 12, in which the trajectories T1, T2, T3 of three reference points P1, P2, P3 of the jaw are displayed.

With reference to Figure 4, in a subsequent stage, or contextual to the previous one, the display device 12 shows a graphic representation of three Cartesian planes X-Y, X-Z, Y-Z (sagittal, frontal and transverse) where at least one line is displayed in each plane T1-L, T1-F, T1-S which represents the movement trajectory of a reference point P1 of the jaw in the respective Cartesian plane.

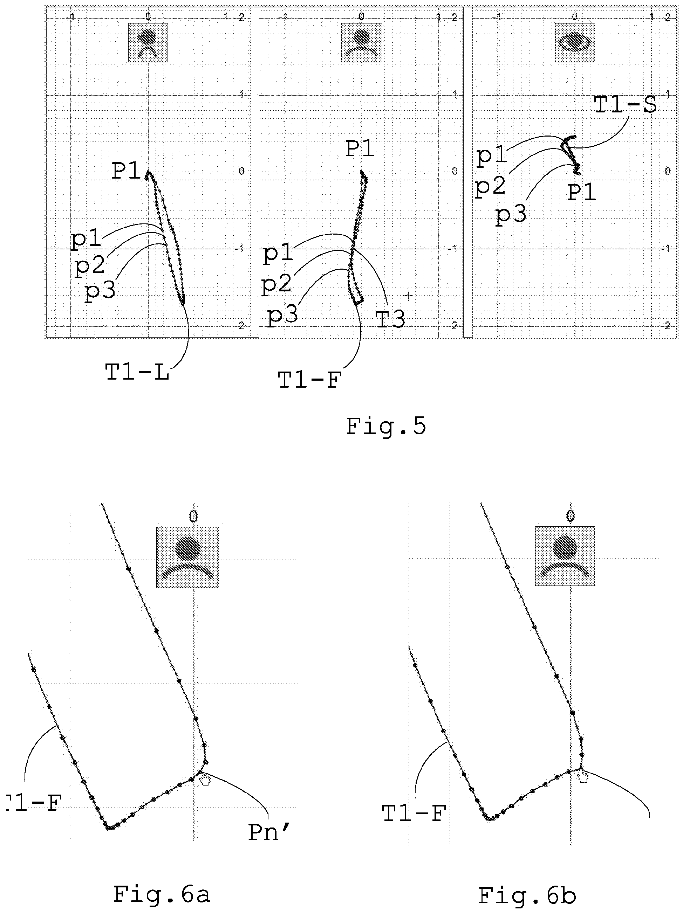

With reference to Figure 5, the display device 12 shows three representations of three Cartesian planes X-Y, of coordinates xₙ-yₙ, xₙ-zₙ, yₙ-zₙ and at least one line T1-L, T1-F, T1-S which interpolates said points N points p.

Said graphic representation of Figure 5 may be generated upon command given to the computer system by the operator or it may be displayed automatically to replace the representation of Figure 4.

It is possible to intervene on this representation of Figure 5 by modifying the position of one or more points p on one or more Cartesian planes to simulate a corrective intervention.

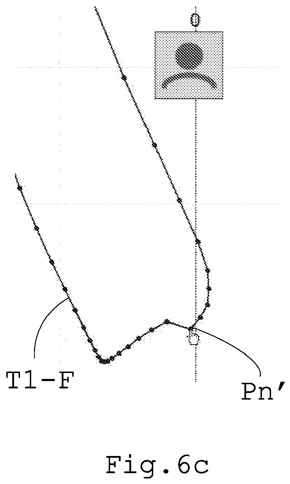

According to a preferred embodiment, through the input device 14, for example a mouse or the like, it is possible to drag a point p in the Cartesian plane from its original coordinate to a new coordinate, as shown in Figures 6a to 6c with reference to a point p^{n'} in the frontal plane X-Z.

Once the dragging of the point or points involved has been completed, the computer system 10 calculates the new Cartesian coordinates for each moved point p and for each Cartesian plane X-Y, X-Z, Y-Z.

At the same time, the computer system recalculates a new updated interpolation function of the N points whose graphic representations are updated as new lines T1-L, T1-F, T1-S shown on the display device 12.

After the corrective intervention made to the virtual trajectories displayed on the display device 12, the computer system 10 may send the corresponding updated data set D to the simulator device 60.

In this way, the dentist may evaluate the effect of the correction made through the real or virtual simulation of the device.

The stages relating to the correction of the position of the points p in the Cartesian planes X-Y, X-Z, Y-Z, to the recalculation of the updated coordinates of the points, to the transmission of the data set D to the simulator device and to the simulation of the movement with the corrective intervention may be repeated until the dentist has identified the most suitable extent of corrective intervention.

As is apparent, the apparatus according to the present invention allows said simulations of the corrective interventions to be generated starting from the real data of the person, thus reducing the operations of adapting the corrective intervention (prosthesis, orthodontic appliance, etc.).

The apparatus according to the present invention is also very practical and intuitive to use for the dentist or other operator, significantly reducing the time necessary to complete the study and evaluation of the corrective intervention.

## Claims

1. An apparatus (1) for simulating the movements of the temporomandibular joint, comprising:
- a computer system (10) configured to generate data sets (D) representative of a movement of a person's jaw, said data being in the form of a set of Cartesian coordinates (xₙ-yₙ, xₙ-zₙ, yₙ-zₙ) on at least one, two or three Cartesian reference planes (X-Y, X-Z, Y-Z);
- a simulator device (60) configured to receive from the computer system (10) the data set (D) representative of said movement of a person's jaw and to generate a simulation of said movement;
wherein the computer system (10) comprises at least one display device (12) configured to generate, starting from the data set (D) representative of the movement of the jaw, a computerized graphic representation on at least one Cartesian plane (X-Y, X-Z, Y-Z) of a displacement trajectory of a reference point (P1, P2, P3) of the jaw, said graphic representation comprising N points (p) of coordinates (xₙ-yₙ, xₙ-zₙ, yₙ-zₙ) and a line representative of an interpolation function of said points,
wherein the computer system (10) comprises an input device (14), operatively connected to the display interface (12), configured to impart, through said display interface (12), a manual command to move at least one point pⁿ (xₙ-yₙ, xₙ-zₙ, yₙ-zₙ) on at least one Cartesian axis (X-Y, X-Z, Y-Z),
the computer system (10) being furthermore configured to recalculate the coordinates of said at least one moved point pⁿ and to generate a new data set (D) representative of the movement of the jaw comprising updated Cartesian coordinates (xₙ-yₙ, xₙ-zₙ, yₙ-zₙ) and, preferably, a new interpolation function of the updated coordinate points.

2. The apparatus (1) according to claim 1, wherein the data set (D) representative of the movement of a person's jaw generated by the computer system (10) comprises Cartesian coordinates (xₙ-yₙ, xₙ-zₙ, yₙ-zₙ) on three Cartesian reference planes (X-Y, X-Z, Y-Z), corresponding to the sagittal, frontal and transverse planes, and wherein the display device (12) is configured to generate respective computerized graphic representations of respective displacement trajectories of the reference point (P1 P2, P3) on said three Cartesian planes (X-Y, X-Z, Y-Z).

3. The apparatus (1) according to claim 1 or 2, wherein the input device (14) comprises a mouse or a track pad which are configured to control, through software implemented in the computer system (10), a pointer shown on the display device, or a touch screen integrated into the display device.

4. The apparatus (1) according to any one of the dependent claims, wherein the interpolation function of the N points (p) of coordinates (xn-yn, xn-zn, yn-zn), representative of the displacement trajectory of the reference point (P1, P2, P3) of the jaw, is a spline, preferably a cubic spline.

5. The apparatus (1) according to any one of the dependent claims, wherein the computer system (10) is configured to send to the simulator device (60) a data set representative of the real movement of a person's jaw corresponding to or based on the coordinates of the N points (p) of the movement trajectory.

6. The apparatus (1) according to any one of the dependent claims, comprising an apparatus (50) for recording and tracking the movements of a person's jaw comprising a vision system and a computer system configured to extrapolate three-dimensional trajectories of the jaw movement.

7. The apparatus (1) according to any one of the dependent claims, wherein the kinematic trajectories acquired through the acquisition system (50) are represented graphically as lines joining the points (p) with three-dimensional coordinates xn-yn-zn with respect to a reference system X, Y, Z.

8. The apparatus (1) according to any one of the dependent claims, wherein the reference point (P1, P2, P3) of the jaw is the barycentre of the jaw, or is a point located on one of the teeth of the jaw.

## Patentansprüche

1. Eine Vorrichtung (1) zur Simulation von Bewegungen des Kiefergelenks, umfassend:
• ein Computersystem (10), das dazu konfiguriert ist, Datensätze (D) zu erzeugen, die eine Bewegung eines Kiefers einer Person repräsentieren, wobei die Daten in Form eines Satzes von kartesischen Koordinaten (xₙ-yₙ, xₙ-zₙ, yₙ-zₙ) auf mindestens einer, zwei oder drei kartesischen Referenzebenen (X-Y, X-Z, Y-Z) vorliegen;
• eine Simulationsvorrichtung (60), die dazu konfiguriert ist, von dem Computersystem (10) den Datensatz (D) zu empfangen, der die Bewegung des Kiefers einer Person repräsentiert, und eine Simulation dieser Bewegung zu erzeugen;
wobei das Computersystem (10) mindestens eine Anzeigevorrichtung (12) umfasst, die dazu konfiguriert ist, basierend auf dem Datensatz (D), der die Bewegung des Kiefers repräsentiert, eine computergenerierte grafische Darstellung mindestens einer Verschiebungstrajektorie eines Referenzpunkts (P1, P2, P3) des Kiefers auf mindestens einer kartesischen Ebene (X-Y, X-Z, Y-Z) zu erzeugen, wobei die grafische Darstellung N Punkte (p) mit Koordinaten (xₙ-yₙ, xₙ-zₙ, yₙ-zₙ) und eine Linie umfasst, die eine Interpolationsfunktion der Punkte repräsentiert;
wobei das Computersystem (10) eine Eingabevorrichtung (14) umfasst, die operativ mit der Anzeigevorrichtung (12) verbunden und dazu konfiguriert ist, über die Anzeigevorrichtung (12) einen manuellen Befehl zum Bewegen mindestens eines Punkts pⁿ (xₙ-yₙ, xₙ-zₙ, yₙ-zₙ) auf mindestens einer kartesischen Achse (X-Y, X-Z, Y-Z) zu übermitteln, wobei das Computersystem (10) ferner dazu konfiguriert ist, die Koordinaten des mindestens einen bewegten Punkts pⁿ neu zu berechnen und einen neuen Datensatz (D) zu erzeugen, der die Bewegung des Kiefers repräsentiert und aktualisierte kartesische Koordinaten (xₙ-yₙ, xₙ-zₙ, yₙ-zₙ) und vorzugsweise eine neue Interpolationsfunktion der aktualisierten Koordinatenpunkte umfasst.

2. Die Vorrichtung (1) nach Anspruch 1, wobei der von dem Computersystem (10) erzeugte Datensatz (D), der die Bewegung eines Kiefers einer Person repräsentiert, kartesische Koordinaten (xₙ-yₙ, xₙ-zₙ, yₙ-zₙ) auf drei kartesischen Referenzebenen (X-Y, X-Z, Y-Z) umfasst, die den sagittalen, frontalen und transversalen Ebenen entsprechen, und wobei die Anzeigevorrichtung (12) dazu konfiguriert ist, jeweilige computergenerierte grafische Darstellungen jeweiliger Verschiebungstrajektorien des Referenzpunkts (P1, P2, P3) auf den drei kartesischen Ebenen (X-Y, X-Z, Y-Z) zu erzeugen.

3. Die Vorrichtung (1) nach Anspruch 1 oder 2, wobei die Eingabevorrichtung (14) eine Maus oder ein Trackpad umfasst, die dazu konfiguriert sind, über eine in dem Computersystem (10) implementierte Software einen auf der Anzeigevorrichtung dargestellten Zeiger oder einen in die Anzeigevorrichtung integrierten Touchscreen zu steuern.

4. Die Vorrichtung (1) nach einem der abhängigen Ansprüche, wobei die Interpolationsfunktion der N Punkte (p) mit Koordinaten (xn-yn, xn-zn, yn-zn), die die Verschiebungstrajektorie des Referenzpunkts (P1, P2, P3) des Kiefers repräsentieren, eine Spline-Funktion, vorzugsweise eine kubische Spline-Funktion, ist.

5. Die Vorrichtung (1) nach einem der abhängigen Ansprüche, wobei das Computersystem (10) dazu konfiguriert ist, an die Simulationsvorrichtung (60) einen Datensatz zu senden, der die tatsächliche Bewegung eines Kiefers einer Person repräsentiert, der den Koordinaten der N Punkte (p) der Bewegungstrajektorie entspricht oder auf diesen basiert.

6. Die Vorrichtung (1) nach einem der abhängigen Ansprüche, umfassend eine Vorrichtung (50) zur Aufzeichnung und Verfolgung von Bewegungen eines Kiefers einer Person, die ein visuelles System und ein Computersystem umfasst, das dazu konfiguriert ist, dreidimensionale Bewegungstrajektorien des Kiefers zu extrapolieren.

7. Die Vorrichtung (1) nach einem der abhängigen Ansprüche, wobei die kinematischen Trajektorien, die durch das Erfassungssystem (50) erfasst werden, grafisch als Linien dargestellt werden, die die Punkte (p) mit dreidimensionalen Koordinaten xn-yn-zn in Bezug auf ein Referenzsystem X, Y, Z verbinden.

8. Die Vorrichtung (1) nach einem der abhängigen Ansprüche, wobei der Referenzpunkt (P1, P2, P3) des Kiefers der Schwerpunkt des Kiefers ist oder ein Punkt, der sich auf einem der Zähne des Kiefers befindet.

## Revendications

1. Un appareil (1) pour simuler les mouvements de l'articulation temporo-mandibulaire, comprenant :
• un système informatique (10) configuré pour générer des ensembles de données (D) représentatifs d'un mouvement de la mâchoire d'une personne, lesdites données se présentant sous la forme d'un ensemble de coordonnées cartésiennes (xₙ-yₙ, xₙ-zₙ, yₙ-zₙ) sur au moins un, deux ou trois plans de référence cartésiens (X-Y, X-Z, Y-Z);
• un dispositif simulateur (60) configuré pour recevoir dudit système informatique (10) l'ensemble de données (D) représentatif dudit mouvement de la mâchoire d'une personne et pour générer une simulation dudit mouvement;
dans lequel le système informatique (10) comprend au moins un dispositif d'affichage (12) configuré pour générer, à partir de l'ensemble de données (D) représentatif du mouvement de la mâchoire, une représentation graphique informatisée sur au moins un plan cartésien (X-Y, X-Z, Y-Z) d'une trajectoire de déplacement d'un point de référence (P1, P2, P3) de la mâchoire, ladite représentation graphique comprenant N points (p) de coordonnées (xₙ-yₙ, xₙ-zₙ, yₙ-zₙ) et une ligne représentative d'une fonction d'interpolation desdits points,
dans lequel le système informatique (10) comprend un dispositif d'entrée (14), relié fonctionnellement à l'interface d'affichage (12), configuré pour impartir, via ladite interface d'affichage (12), une commande manuelle permettant de déplacer au moins un point pⁿ (xₙ-yₙ, xₙ-zₙ, yₙ-zₙ) sur au moins un axe cartésien (X-Y, X-Z, Y-Z),
le système informatique (10) étant en outre configuré pour recalculer les coordonnées dudit au moins un point déplacé pⁿ et pour générer un nouvel ensemble de données (D) représentatif du mouvement de la mâchoire comprenant des coordonnées cartésiennes mises à jour (xₙ-yₙ, xₙ-zₙ, yₙ-zₙ) et, de préférence, une nouvelle fonction d'interpolation des points de coordonnées actualisés.

2. L'appareil (1) selon la revendication 1, dans lequel l'ensemble de données (D) représentatif du mouvement de la mâchoire d'une personne, généré par le système informatique (10), comprend des coordonnées cartésiennes (xₙ-yₙ, xₙ-zₙ, yₙ-zₙ) sur trois plans de référence cartésiens (X-Y, X-Z, Y-Z), correspondant respectivement aux plans sagittal, frontal et transversal, et dans lequel le dispositif d'affichage (12) est configuré pour générer des représentations graphiques informatisées respectives de trajectoires de déplacement respectives du point de référence (P1, P2, P3) sur lesdits trois plans cartésiens (X-Y, X-Z, Y-Z).

3. L'appareil (1) selon la revendication 1 ou 2, dans lequel le dispositif d'entrée (14) comprend une souris ou un pavé tactile configurés pour commander, via un logiciel implémenté dans le système informatique (10), un pointeur affiché sur le dispositif d'affichage ou un écran tactile intégré audit dispositif d'affichage.

4. L'appareil (1) selon l'une quelconque des revendications dépendantes, dans lequel la fonction d'interpolation des N points (p) de coordonnées (xn-yn, xn-zn, yn-zn), représentative de la trajectoire de déplacement du point de référence (P1, P2, P3) de la mâchoire, est une spline, de préférence une spline cubique.

5. L'appareil (1) selon l'une quelconque des revendications dépendantes, dans lequel le système informatique (10) est configuré pour envoyer au dispositif simulateur (60) un ensemble de données représentatif du mouvement réel de la mâchoire d'une personne correspondant ou basé sur les coordonnées des N points (p) de la trajectoire de mouvement.

6. L'appareil (1) selon l'une quelconque des revendications dépendantes, comprenant un dispositif (50) d'enregistrement et de suivi des mouvements de la mâchoire d'une personne, comprenant un système de vision et d'un système informatique configuré pour extrapoler des trajectoires tridimensionnelles du mouvement de la mâchoire.

7. L'appareil (1) selon l'une quelconque des revendications dépendantes, dans lequel les trajectoires cinématiques acquises par le système d'acquisition (50) sont représentées graphiquement sous la forme de lignes reliant les points (p) de coordonnées tridimensionnelles xn-yn-zn par rapport à un système de référence X, Y, Z.

8. L'appareil (1) selon l'une quelconque des revendications dépendantes, dans lequel le point de référence (P1, P2, P3) de la mâchoire est le barycentre de la mâchoire ou un point situé sur l'une des dents de la mâchoire.
